# EUROPEAN PATENT APPLICATION

(11) **EP 2 386 355 A1**
(43) Date of publication of application: **16.11.2011**
(21) Application number: 10162499.7
(22) Date of filing: 11.05.2010
(51) Int. Cl.: B01J 31/00, B01J 23/44, B01J 23/50, B01J 23/89, B01J 35/00, B01J 35/02, B01J 37/02, B22F 1/02, B22F 9/02, B22F 1/00, C07C 1/26, A62D 3/37, C02F 1/70

(54) **Metallic alloys with microbiological component and catalytic properties.**

(71) Applicant: Biorem Engineering SARL, 62780 France (FR)
(72) Inventor: Lakaye, Frédéric, 62780, France (FR); De Windt, Wim, 9040, Sint-Amandsberg (BE)
(74) Representative: Vandeberg, Marie-Paule L.G.

(57) **Abstract**

This invention concerns a new material with enhanced catalytic properties, produced by mechanical alloying of microbially encapsulated metallic (or zerovalent) nanoparticles with a catalyst. The bioencapsulation ensures a maximized contact area for molecular restructuring, since the microbial biomass can prevent agglomeration during the mechanical alloying process. The resulting product is a metallic alloy with at least 1 % of the material dry weight comprising microbial biomass and with enhanced catalytic properties.

## Description

### Domain of the Invention

New materials with enhanced catalytic properties were produced by mechanical alloying of microbially encapsulated metallic (or zerovalent) nanoparticles with a catalyst. The bioencapsulation ensures a maximized contact area for molecular restructuring, since the microbial biomass acts as binder and process control agent during the mechanical alloying process. The resulting product is a metallic alloy with at least 1 % of the material dry weight comprising microbial biomass and with enhanced catalytic properties when compared to the sum of the actions of both components before alloying. The mechanical alloying results in a solid-solid reaction between the catalyst and the co-catalyst(s) and at least one of these comprises microbially encapsulated nanoparticles.

Potential applications are situated in the fields of groundwater remediation, soil or sediment remediation, water treatment, off-gas treatment, components of fuel cells, automotive catalytic convertors, and many other fields of catalysis.

### Prior Art

A heterogeneous catalyst is present in a different aggregation state than the reactants. Most often, the reactants are in a gaseous or liquid phase and the reaction takes place at the surface of a solid catalyst particle, that does not partake in the reaction. It is well known in the art that the spectrum of reactions that are catalyzed by a heterogeneous catalyst can be widened by adding a second (or third) component or co-catalyst to the system. When the catalyst and the co-catalyst(s) contain at least one metallic component, an alloy can be formed. According to Ponec and Bond (1995), the term "alloy" can be used to describe any metallic system containing two or more components, irrespective of their intimacy of mixing or the precise manner in which their atoms are disposed. On this account, alloys can be formed from a metal and a clearly non-metal, or two or more metals forming a continuous series of solid solutions over the whole concentration range.

Bimetallic catalysts are most often produced by complex and expensive technologies such as controlled chemical deposition of one metal onto another (Lin et al., 2004) or by impregnation of a support into a metallic salt solution, microwave irradiation (Lingaiah et al., 2001) or by arc melting (Choi et al., 2009).

While it is true that combinations of bimetals with living bacteria have been described in literature for a combined biological and chemical catalysis process, for example in He et al., 2009 where palladium-coated iron and an anaerobic bacterium catalyze the dechlorination of pentachlorobiphenyl, this is simply a sequential combination of a chemical and biological process, when both processes are separated in space and time. The drawback of such a simple sequential combination is that the co-catalytic effect of one component on the other component cannot be achieved due to the fact that both components are separated in space (absence of mixed sites).

In some other papers, the mechanical alloying of metallic phases in a mill is described, such as in DeVor et al., 2009, however here both metallic phases are present in a powder form, without suitable capping agent and without the specific presence of one of the components associated with a microbial binder. As a result, cake formation and agglomeration will typically take place during the mechanical alloying process, which results in an end-product with less-than-optimal reactivity since the so-called "mixed-sites" (very reactive bimetallic spots) are either not formed or the interaction between the alloyed components is too weak.

### General Description of the Invention

In this invention, it is described that the bacterial biomass acts as an especially suitable capping agent for zerovalent metallic nanoparticles (metallic nanoparticles which may be produced by microbial reduction processes) and can be further used as a component for mechanical alloying with another catalytic component in order to produce one final end-product: a unique and novel form of alloy where the microbial biomass is stabilizing and binding the nanoparticles, as well as functioning as a process control agent during the mechanical alloying, preventing cake formation and agglomeration.

The object of this invention is to modify the reaction rate per unit of surface area (turnover frequency) of a metallic catalytic component by mechanical alloying this catalytic component with at least one other component, where the at least one of the components comprises (micro-) biologically encapsulated metallic nanoparticles. During the mechanical alloying, the different crystallographic planes at the catalyst surface are exposed and made imperfect, one component is alloyed into the other component by a method chosen from compression-shear stressing, impact stressing, stressing in shear flow and/or stressing by a thermal field, a chemical field or an electrical field, ball milling. A preferred mechanical alloying methodology was proven to be ball milling in combination with spraydrying.

### Detailed Description of the Invention

By ball milling a microbiologically encapsulated catalytic component together with a second and/or third co-catalytic component, which may also be microbiologically encapsulated, small metallic nanoparticles occupy preferential sites of a given surface topology, and an alloy with enhanced catalytic turnover rate is produced. The microbial biomass stimulates mixed site formation during ball milling, since it functions as a 3D scaffold that binds catalytic component and co-catalytic component(s). In this way, local mechanical alloying at nanoscale results in very reactive at least bimetallic spots or "mixed-sites" at the surface of the catalytic component, resulting in surface layer reconstructing, and in enhanced catalytic reactivity due to electronic interaction between the different alloyed components. A mixed-site is an active site where all components of the alloys participate in the catalytic transformation.

A preferred methodology for microbiologically encapsulating catalytic or co-catalytic nanoparticles is by microbially mediated bioreduction of salts of catalytic metals such as Pd(II) salts or Ag(I) salts and concomitant precipitation of the reduced metals, often in zerovalent state, onto or into the microbial biomass (glycocalyx, S-layer, polysaccharides, or other outer cell constituents of the microbial cell).

The influence of a co-metal on catalyst performance is crucially affected by: (i) the degree of intimate interaction between the two components in the same aggregates; and (ii) the chemical state of the co-metal.

These prerequisites pose problems in the production of such catalytic alloys at industrial scale, since (i) intimate interaction at molecular scale is difficult to achieve, and (ii) most often co-catalysts are doped onto the heterogeneous catalyst surface by deposition processes, like deposition of an organometallic complex or inorganic salt, whereby the co-catalyst is in a non-zerovalent state. This poses problems when the ideal chemical state of the co-metal is the zerovalent metallic state.

In the production process of the applicant, these problems have been overcome as follows: (i) the catalyst and co-catalyst are already in the metallic state before alloying, and (ii) the intimate interactions are favoured by the nano-scale of one or both of the components, and (iii) by the binding of the catalytic with the co-catalytic components in a catalytically inert biomatrix which favours the mechanical alloying process.

In a preferred embodiment for the production of microbially deposited zerovalent metallic nanoparticles, the microbial biomass acts both as a reducing agent and as a capping agent for the metallic nanoparticles after reduction into the zerovalent state.

The at least one component comprising metallic nanoparticles encapsulated (or capped) in a microbial biomass (and alloyed to the surface of an other component) is preferably chosen from palladium, platinum, silver, gold, ruthenium, nickel, zinc, vanadium, cerium or copper.

The microbial biomass is obtainable from bacteria, algae or fungi.

More particularly, the microbial biomass can be chosen from proteins, DNA, glycoproteins, glycocalyx or biopolymers.

The bacteria are preferably chosen from the genera *Shewanella* sp., *Lactobacillus* sp., *Geobacter* sp., *Escherichia* sp., *Pseudomonas* sp., *Bacillus* sp., *Lactococcus* sp. or *Magnetospirillium* sp. A more preferred species is *Shewanella oneidensis* MR-1.

The catalytic composition comprises preferably between 1 and 95 dry weight percent of microbial biomass, more preferentially between 1 and 20 %. The ratio of one component to the other can be chosen between 1:100 and 1:2. The metallic nanoparticles stabilized by microbial biomass have preferably a size smaller than 100 nm, and more preferentially individual nanoparticles are between 1 and 10 nm.

The at least (co-)catalytic second component is chosen preferentially from palladium, iron, platinum, gold, aluminium oxide, ruthenium, copper, silver, silver oxide, magnesium, zinc, titanium dioxide, vanadium, cerium or nickel.

This invention also relates to a method for producing a catalytic composition, comprising a mechanical alloying process of at least one component comprising metallic catalytic nanoparticles encapsulated in microbial biomass alloyed to the surface of at least a second component.

In a preferred embodiment, this at least second component is a (co-)catalytic component.

Preferably, the mechanical alloying process comprises a step chosen from mechanofusion, dry impact blending, ball milling, spray drying, impaction coating, ultra-fine grinding or any combination of aforementioned operations, executed in a planetary ball mill, hybridizer, pin mill, jet mill, drum mill, ultrasonic mill, electromagnetic mill, elliptical rotor mixer, pearl mill or attrition mill.

In another preferred embodiment, either the at least one component, the at least second component or both are in dry powder form.

For some applications, one of these components or both may be dispersed in a liquid phase, such as an alcohol or a volatile alkane, or in a slurry with water or other water-based solvents.

Generally, the at least one component comprising metallic nanoparticles is obtainable by a microbial biomass reduction step.

In present invention, it was surprisingly found that by spray-drying a microbially deposited metallic nanoparticle slurry into a powder, 3D-structures were formed with a particular surface topology. By ball milling these "biostabilized" structures containing a catalytic nano-sized component with a (co-)catalyst in powder or slurry, alloys are formed with modified physicochemical properties, such as for example new catalytic properties, different surface topology (characterized by kinks, edges, cracks, corners and low index planes), new magnetic properties, new conductive properties, new antimicrobial properties and amphiphilic properties. The microbial biomass does not prevent the alloying and even has a positive effect on the mechanical alloying process, likely because the biomass brings the individual nanoparticles in the right position for mixed site formation during ball milling, and by preventing cake formation and agglomeration.

In the following examples, proof is given for increased catalytic properties of the metallic alloys with microbiological component thus produced. It was found that the conversion rate for hydrodechlorination of chlorinated solvents and polychlorinated biphenyl was increased, and more dechlorinated end-products were formed by catalysis with the thus produced metallic alloys.

This process which has been developed at laboratory scale is readily upscalable.

### Example I. Preparation of a palladium-iron-biomass alloy

Catalytic zerovalent iron powder was obtained from Alfa Aesar (Karlsruhe, Germany) with a D50 < 10 µm.

Biologically precipitated palladium nanoparticles were produced in a 20 m³ fermentor reaction vessel. The catalytic palladium nanoparticles were produced and then stabilized in the glycocalyx of *Shewanella oneidensis* MR-1 bacterial cells. *Shewanella oneidensis* MR-1 was fermented in 20 m³ Luria Bertani fermentation broth during 24 hours, and a total of 501 kg concentrated fermentate was obtained by centrifugation, containing 3.87 % dry matter. Three hundred twenty (320) kg of this concentrated fermentate was added to 24.4 m³ of M9 mineral salt solution containing 25 mM sodium formate. An optical density of 1.16 (OD610) was obtained in the reaction vessel containing the mineral salt solution. To this solution, 7300 grams of palladium (Pd²⁺) was added as sodium tetrachloropalladate salt. The pH of the solution was 7.2 and the suspension contained about 12.2 grams of dry weight per litre. After 24 hours of reaction, the solution has turned completely black, indicative of a bioreductive process where Pd²⁺ was reduced to Pd⁰ by *Shewanella oneidensis* MR-1 cells.

The bacterial cells containing the metallic palladium nanoparticles were harvested by centrifugation, washed with a salt solution and concentrated by centrifugation into a water-based slurry. The individual size of the palladium nanoparticles was examined by Transmission Electron Microscopy, as described in Example 2.

This palladium containing water-based slurry was then further processed by spray-drying and atomized into a powder. Spray drying was done according the following procedure: the slurry was continuously mixed to guarantee the equal dispersion of the palladium before spray-drying. The atomization parameters are described in Table 1 .

**Table 1. Process parameters used during the atomization of the palladium containing water-based slurry.**

| **Parameter** | |
|---|---|
| Product | Nanopalladium in biomass slurry |
| Machine type | Niro Production Minor |
| Temp inlet | +/- 180 - 190°C |
| Temp outlet | +/- 60 - 70 °C |
| Time | 5h15 |
| Yield | 93 % |

After the atomization process, a palladium containing powder was obtained that was further examined by Electron Microscopy as described in Example 3.

A total of 95 grams of zerovalent iron powder was added together with 12 grams of spray-dried nanoparticles stabilized in *Shewanella oneidensis* microbial biomass containing 5 grams of metallic palladium particles, to one of the milling cups of a Retsch PM 400 ball milling station. The powders were ball milled during 45 minutes under nitrogen gas atmosphere, to prevent oxidation processes.

The resulting ball milled powder was found to be magnetic.

### Example II. Determination of Particle Size of Nanopalladium in microbial biomass by means of Transmission Electron Microscopy

In order to prepare thin sections for analysis by TEM, pellets of bacteria were fixed in 0.1 M of a cacaodylate buffer (pH 7.4) containing 2.5% glutaraldehyde and 2% formaldehyde, and embedded in 3% low melting agarose (from Difco Laboratories, Detroit, Mich., USA). These samples were post-fixed in 1% osmium tetroxide. Between and after fixation steps, samples were washed with distilled water. Afterwards, samples were dehydrated in increasing concentrations of ethanol and, finally, in anhydrous propylene oxide. After embedding in Epon-Spurr medium, the specimen blocks were trimmed with a TM60 trimming unit (from Reichert-Jung A. G., Vienna, Austria) to obtain a cutting face of 0.5×1 mm2-1×2 mm2, and ultra-thin sections in the gold to mat silver interference color range were cut using the Ultracut microtome (from Reichert-Jung A.G., Vienna, Austria). The sections were brought on pioloform and carbon coated copper grids (200 mesh). Once this was done, thin sections were stained with 2% uranyl acetate and then with lead citrate to determine the ultra-structure of the cells. Chemicals and grids were obtained from Agar Scientific (Stansted, United Kingdom). Imaging was performed with a EM208S transmission electron microscope (from FEI, Eindhoven, the Netherlands) operating at 80 kV.

TEM images (not shown) have been obtained for the nanopalladium particles resulting from the production in *Shewanella oneidensis* sp. as described in example 1. These images confirm that spherical nanopalladium particles were obtained in the composition in the form of precipitates on the bacterial cell-surface.

Particle sizes for nanoPd resulting from production in *Shewanella oneidensis* MR-1, where a ratio of biomass dry weight to palladium of 2.89 : 1 was obtained :
The diameter ranged from 3 nm to 56 nm with an average of 11 nm.

### Example III. SEM study of morphology of Nanopalladium in microbial biomass, iron and FePd alloy

By means of a JSM6100 Scanning Electron Microscope, dry samples of nanopalladium produced in *Shewanella oneidensis* MR-1 after spray-drying, metallic iron powder, and ball-milled FePd alloy were examined on Si wafers.

The Fe particles (Alfa Aesar, Germany) has a D50 < 10 µm and were spheroidal in shape. The spray-dried nanopalladium in microbial biomass consisted of individual spheroidal particles with an irregular surface curvature of about 1-2 microns in size. This powder was found to have a spheroidal micron-sized structure with a surface topology characterized by kinks, edges, cracks, corners and low index planes.

After ball-milling, an alloy was obtained where the individual particles had an average diameter of about 1.5 micrometer, spheroidal in shape. No individual spray-dried spheroidal bodies could be detected after ball-milling.

### Example IV: SEM-EDX analysis of FePd

Energy Dispersive X-ray (EDX) analysis of an alloy of metallic Fe powder with palladium nanoparticles produced in microbial biomass as obtained in example 1 was performed with a JSM6100 Scanning Electron Microscope with EDX detector (available from JEOL USA, Inc.) with a resolution corresponding to an incident energy of 20.0 keV. Analysis results are listed in Table 2 (both as weight % and atomic %) and clearly demonstrate the presence mainly of organic matter (due to the high content of carbon), palladium and iron, the combination of which amounts to about 98.5 weight % of the dry matter. The rest of the ball-milled product consisted of trace elements Na, Si and P, mostly due to mineral residues from the dried biological matrix.

**Table 2. Energy-Dispersive X-ray Spectrum of FePd alloy obtained by ball-milling Fe powder with spray-dried nanopalladium in Shewanella oneidensis MR-1 biomass.**

| **Element** | **Weight %** | **Atomic %** |
|---|---|---|
| C | 16.34 ± 4.22 | 46.69 ± 7.70 |
| Na | 0.86 ± 0.56 | 1.27 ± 0.76 |
| Si | 0.35 ± 0.18 | 0.42 ± 0.20 |
| P | 0.28 ± 0.18 | 0.31 ± 0.19 |
| Pd | 1.44 ± 0.73 | 0.47 ± 0.23 |
| Fe | 80.73 ± .05 | 50.84 ± 8.34 |

### Example V. The use of a palladium-iron-biomass alloy

Catalytic reactivity tests were performed where the ball milled alloy was compared to the two precursor powders in terms of hydrodechlorination catalysis potential for a chlorinated solvent, 1,1,1-trichloro ethane (1,1,1-TCA), and for dechlorination of a polychlorinated biphenyl congener (PCB 118).

The tests with 1,1,1-TCA were executed in 100 ml closed penicillin bottles with Viton stoppers, containing 50 ml milliQ water that was de-aerated and into which 10 mg/L 1,1,1-TCA had been spiked from a 1,1,1-TCA stock solution in methanol. De-aeration was done by flushing for example with argon gas, no oxygen was present in the water volume. The headspace of the penicillin bottles contained 100 % Ar gas at the start of the experiment. All experiments were set up in duplicate. The powders were added to the respective penicillin bottles in the following concentration: 2000 mg/l iron powder (Fe), 250 mg/l biomass containing 120 mg palladium nanoparticles (Pd) and 2140 mg/l biomass alloy (FePd). Assays were incubated at 28 °C.

Depending on the chlorinated component, analyses were done in samples of the headspace or in the water phase. Ethane, ethene and 1,1,1-TCA were measured in the headspace by GC-FID analysis (Varian).

For 1,1,1-TCA a capillary column (Varian FactorFour Low Bleed VF624 ms, 30 m x 0.25 mm) was used. The temperature of the injector was 200 °C with a split ratio of 5. The setup of the temperature program was 30 °C for 2 minutes, increasing to 40 °C at 1 °C/min and finally to 105 °C at 5 °C/min. The carrier gas was helium at a flow rate of 3 ml/min. The temperature of the FID detector was kept at 250 °C. The detection limit was 10 µg/l.

Ethane and ethene were separated on a Varian Poraplot Q column, 25 m x 0.53 mm. GC injections were made at 30 °C. This temperature was maintained for 3 min, followed by an increase to 80 °C at 30 °C/min and finally to 150 °C at 10 °C/min. The temperature of the injector was 200 °C with a split ratio of 5. The carrier gas was He at a flow rate of 10 ml/min. The temperature of the FID detector was 240 °C.

The tests with PCB 118 were executed in 100 ml closed penicillin bottles with Viton stoppers, containing milliQ 30 ml water. PCB 118 was added from a methanol stock solution to a final concentration of 1 mg/l. All experiments were set up in duplicate. The powders were added to the respective penicillin bottles in the following concentration: 2000 mg/l iron powder (Fe), 250 mg/l biomass containing 120 mg palladium nanoparticles (Pd) and 2140 mg/l biomass alloy (FePd). The suspensions were anaerobised by consecutive cycles of underpressure and Ar overpressure. The anoxic headspace was finally saturated with 100 % H₂ gas in the test with Pd and filled with 100 % Ar gas in all other tests.

PCBs were analyzed with a gas chromatograph (GC), Varian 3800 (Varian, Middelburg, The Netherlands) equipped with an electron capture detector (ECD). N₂ was used as carrier gas at a flow rate of 1,3 ml min⁻¹. One µl of the extracted sample was injected splitless. For detection of PCBs and their dechlorination products, temperature was held for 2 min at 100 °C, increased to 160 °C with a gradient of 15 °C min⁻¹, followed by 5 °C min⁻¹ to 270 °C and held at 270 °C for 10 min. The pressure in the capillary column was 1,09 kg/cm².

The results of these reactivity tests are shown in Tables 3 and 4.

**Table 3. Concentration of 1,1,1-Trichloro ethane (1,1,1-TCA) and dechlorination products ethane and ethane in function of incubation time in the presence of 3 different catalytic compositions : Zerovalent iron powder (Fe), Microbially precipitated palladium nanoparticles (Pd), and an Iron-palladium-biomass alloy (FePd).**

| **Time of incubation (h)** | | | | | | | | | |
|---|---|---|---|---|---|---|---|---|---|
| | **0** | | | **6** | | | **72** | | |
| **Catalyst** | 1,1,1-TCA | Ethane | Ethene | 1,1,1-TCA | Ethane | Ethene | 1,1,1-TCA | Ethane | Ethene |
| **Fe** | 100 | 0 | 0 | 125 | 2 | 0 | 100 | 5 | 0 |
| **Pd** | 80 | 0 | 0 | 75 | 0 | 0 | 70 | 0 | 0 |
| **FePd** | 75 | 0 | 0 | 43 | 12 | 0 | 0 | 25 | 0 |

From the results in Table 3, it can be seen that zerovalent iron powder and microbially produced palladium nanoparticles are an ineffective catalyst for 1,1,1-TCA dechlorination, whereas the FePd biomass alloy results in complete removal of 1,1,1-TCA within 72 hours and with formation of significant amounts of ethane in the headspace, indicative of complete and effective hydrodechlorination. An increase of 1,1,1-TCA with time is most probably due to a lack of equilibrium between liquid and gaseous phases at the time of analysis.

**Table 4. Concentration of PCB 118 in function of time in the presence of 2 different catalytic compositions : Microbially precipitated palladium nanoparticles under H₂ atmosphere, and an Iron-palladium-biomass alloy under Ar atmosphere.**

| | **Time of incubation (h)** | | |
|---|---|---|---|
| **Catalyst** | **0** | **6** | **72** |
| **Pd** | 1000 | 630 | 380 |
| **Fe** | 1000 | - | 1050 |
| **FePd** | 1000 | 420 | 180 |

From the results of Table 4, it can be seen that removal of PCB 118 by hydrodechlorination was more effective with the FePd biomass alloy than with the biomass encapsulated palladium nanoparticles, even when the nanoparticles were saturated with molecular hydrogen gas and no H₂ was added to the FePd biomass alloy. The reason is probably the more effective hydride formation on the palladium surface due to the presence of iron in the FePd "mixed sites", according to the following reactions.

On (anoxic) iron surface the following reaction takes place:

Fe(0) + 2H⁺ → Fe(II) + H₂ (pH<7)

On Palladium surface, the following reaction takes place:

R-Cl + H2 → R-H + HCl

Due to the alloying of Pd and Fe, hydrogen can be more effectively charged onto the palladium surface.

An increase of PCB 118 with time is most probably due to a lack of equilibrium between liquid and gaseous phases at the time of analysis.

### Example VI. Preparation and use of a silver-palladium-biomass alloy

Biologically precipitated palladium nanoparticles were produced as in Example I. Metallic silver was kindly provided by Janssen PMP (Beerse, Belgium).

66 grams of metallic silver powder was added together with 33 grams of spray-dried palladium nanoparticles stabilized in *Shewanella oneidensis* microbial biomass, to one of the milling cups of a Retsch PM 400 ball milling station. The powders were ball milled during 45 minutes.

The catalytic reactivity of the ball milled powder was compared to the two precursor powders in terms of hydrodechlorination catalysis potential for a chlorinated solvent, vinyl chloride.

The tests with vinyl chloride were executed in 100 ml closed penicillin bottles with Viton stoppers, containing 50 ml milliQ water that was de-aerated and into which an equivalent of 50 ppm vinyl chloride in the headspace was dosed. De-aeration had first been done by flushing for example with argon gas, no oxygen was present in the water volume. The headspace of the penicillin bottles contained 100 % Ar gas at the start of the experiment. All experiments were set up in duplicate. The powders were added to the respective penicillin bottles in the following concentration: 625 mg/l silver powder (Ag), 250 mg/l biomass containing 120 mg palladium nanoparticles (Pd) and 770 mg/l biomass alloy (AgPd). Assays were incubated at 28 °C.

Analyses were done by GC-FID with the same protocol as described in Example I.

The results of these reactivity tests are shown in Table 5.

**Table 5. Concentration of Vinyl chloride (VC) and dechlorination products ethane and ethene in function of incubation time in the presence of 3 different catalytic compositions : Zerovalent silver powder (Ag), Microbially precipitated palladium nanoparticles (Pd), and an silver-palladium-biomass alloy (AgPd).**

| **Time of incubation (h)** | | | | | | | | | |
|---|---|---|---|---|---|---|---|---|---|
| | **0** | | | **6** | | | **72** | | |
| **Catalyst** | VC | Ethane | Ethene | VC | Ethane | Ethene | VC | Ethane | Ethene |
| **Ag** | 1750 | 0 | 0 | 1900 | 0 | 0 | 1800 | 50 | 0 |
| **Pd** | 1650 | 0 | 0 | 0 | 4000 | 0 | 0 | 4000 | 0 |
| **AgPd** | 2600 | 0 | 0 | 0 | 12000 | 1000 | 0 | 12000 | 1000 |

From Table 5 it can be seen that zerovalent silver powder (Ag) and microbially produced palladium nanoparticles (Pd) are less effective catalysts for vinyl dechlorination, whereas the AgPd alloy results in complete removal of vinyl chloride within 6 hours and with formation of significant amounts of ethane in the headspace, indicative of complete and effective hydrodechlorination .

Increases of VC with time are most probably due to a lack of equilibrium between liquid and gaseous phases at the time of analysis.

## Claims

1. Catalytic composition comprising at least one component comprising metallic nanoparticles encapsulated in a microbial biomass.

2. Catalytic composition according to claim 1 **characterized in that** at least a second component is present with catalytic properties.

3. Catalytic composition according to claim 1 or 2 **characterized in that** the at least one component is alloyed to the surface of the at least second component.

4. Catalytic composition according to any preceding claim **characterized in that** the one component comprising metallic nanoparticles is chosen from palladium, platinum, silver, ruthenium, nickel, zinc, gold, vanadium, cerium or copper.

5. Catalytic composition according to any preceding claim **characterized in that** the microbial biomass is obtained from bacteria, algae or fungi.

6. Catalytic composition according to claim 5 **characterized in that** the obtained microbial biomass is chosen from proteins, DNA, glycocalyx, glycoproteins or biopolymers.

7. Catalytic composition according to claims 5-6 **characterized in that** the bacteria are chosen from the genera *Shewanella* sp., *Lactobacillus* sp., *Geobacter* sp., *Escherichia* sp., *Pseudomonas* sp., *Bacillus* sp., *Lactococcus* sp. or *Magnetospirillum* sp.

8. Catalytic composition according to any of the claims 2 to 7 **characterized in that** the at least second catalytic component is chosen from palladium, iron, platinum, gold, aluminium oxide, ruthenium, copper, silver, silver oxide, magnesium, zinc, titanium dioxide, vanadium, cerium or nickel.

9. Catalytic composition according to any preceding claim **characterized in that** the metallic nanoparticles have a size smaller than 100 nm.

10. Catalytic composition according to any preceding claim **characterized in that** the catalytic composition comprises between 1 and 95 dry weight percent of microbial biomass.

11. Method for producing a catalytic composition, comprising a mechanical alloying process of at least one component comprising metallic nanoparticles encapsulated in microbial biomass.

12. Method according to claim 11 **characterized in that** at least a second component with catalytic properties is present during the mechanical alloying process.

13. Method according to claims 11-12 **characterized in that** the mechanical alloying process comprises a step chosen from mechanofusion, dry impact blending, ball milling, spray drying, impaction coating, ultra-fine grinding or any combination of aforementioned operations, executed in a planetary ball mill, hybridizer, pin mill, jet mill, drum mill, ultrasonic mill, electromagnetic mill, elliptical rotor mixer, pearl mill or attrition mill.

14. Method according to claims 11-13 **characterized in that** the at least one component, the at least second component or both are in dry powder form.

15. Method according to claims 11-14 **characterized in that** the at least one component, the at least second component or both are dispersed in a liquid phase or slurry.

16. Method according to claims 11-15 **characterized in that** the at least one component comprising metallic nanoparticles is obtained by a microbial biomass reduction step.
